# EUROPEAN PATENT SPECIFICATION

(11) **EP 2 493 892 B1**
(45) Date of publication and mention of the grant of the patent: **01.07.2015**
(21) Application number: 09744572.0
(22) Date of filing: 29.10.2009
(51) Int. Cl.: C07D 498/20, A61K 31/439, A61P 25/28

(54) **QUINUCLIDINE COMPOUNDS AS ALPHA-7 NICOTINIC ACETYLCHOLINE RECEPTOR LIGANDS**
CHINUKLIDINE-DERIVATE ALS ALPHA-7 NIKOTIN ACETYLCHOLIN REZEPTOR LIGANDEN
COMPOSÉ DE QUINUCLIDINE COMME LIGANDS DU RÉCEPTEUR NICOTINIQUE ALPHA-7 DE L'ACÉTYLCHOLINE

(43) Date of publication of application: 05.09.2012
(73) Proprietor: Bristol-Myers Squibb Company, Princeton, NJ 08543 (US)
(72) Inventor: COOK II, James H., Wallingford, CT 06492 (US); MCDONALD, Ivar, M., Wallingford, CT 06492 (US); KING, Dalton, Wallingford, CT 06492 (US); OLSON, Richard, E., Wallingford, CT 06492 (US); WANG, Nenghui, Wallingford, CT 06492 (US); IWUAGWU, Christiana, I., Wallingford, CT 06492 (US); ZUSI, F.,Christopher, Wallingford, CT 06492 (US); MACOR, John, E., Wallingford, CT 06492 (US)
(74) Representative: Reitstötter Kinzebach
(86) International application number: PCT/US2009/062492
(87) International publication number: WO 2011/053292

(56) References cited:
- WO-A-2006/065209
- WO-A-2008/000469
- WO-A-2009/131926

## Description

### BACKGROUND OF THE INVENTION

The disclosure generally relates to compounds of formula I, including their salts, as well as compositions. The compounds are ligands, agonists and partial agonists for the nicotinic α7 receptor and may be useful for the treatment of various disorders of the central nervous system, especially affective and neurodegenerative disorders.

Schizophrenia is a serious mental disorder, affecting approximately 1% of the population. Its progressive course results in major impairment of mental and social functioning and often leads to the development of other pathologies. Susceptibility often runs in families, with both genetic and environmental factors thought to be important. The direct and indirect costs of the disease are estimated in the tens of billion dollars annually in the U.S. alone.

Patients with schizophrenia have an elevated risk of suicide (approximately a 10% lifetime risk). They have a 2.5 fold increase in all-cause mortality, resulting in a 20% lowered life expectancy. The onset of illness can result in cascade of unhealthy lifestyle factors and behaviors that elevate the risk of various conditions and consequently the risk of death.

The onset of schizophrenia is most often in late adolescence or early adulthood, and episodes recur throughout life. The disease is characterized by the expression of three distinct symptom domains: positive, negative and cognitive. Psychotic or positive symptoms include delusions, hallucinations, thought disorder and paranoia. Negative symptoms include negative affect, social withdrawal, and anhedonia. Cognitive dysfunction includes deficits in attention, working memory and executive function. The pathophysiology of schizophrenia is not well understood, however, most experts believe it is a multi-factorial disorder in which biological, genetic and environmental factors play a role. Most current therapies target the dopaminergic system and have resulted in the suggestion that an excess of dopaminergic neurotransmission underlies at least some aspects of schizophrenia. This theory received further support from findings that drugs which increase the levels of dopamine cause psychoses similar to the positive symptoms of the disease. Also, post mortem analysis of brains from schizophrenic patients indicate increased numbers of D2 dopamine receptors. Although newer antipsychotic agents, known as atypical antipsychotics, which are active at several additional neurotransmitter receptors, have been introduced in the past decade, these agents still share efficacy against the D2 dopamine receptor. All currently-used agents also have major limitations. Although positive symptoms are generally reduced in a majority of patients, these drugs do little to relieve the negative symptoms and cognitive deficits that are common and often most debilitating. In addition, antipsychotic agents have a number of unwanted and limiting side effects.

Nicotine is among the few agents which have a positive effect on cognitive function. Many schizophrenics smoke; the rate in patients is 2-4 times that of the general population, and up to 90% in schizophrenics who have been institutionalized do smoke. This smoking habit has been characterized as a form of self-medication.

Nicotinic acetylcholine receptors (nAChR's) are pentameric ligand-gated ion channels which are widely expressed through the central and peripheral nervous system. These channels are fast-desensitizing calcium channels which, when open, increase the intracellular concentration of the Ca⁺⁺ ion. Although there are 12 individual receptors, the most abundant nicotinic receptors in the brain are α4β2 and α7. The α4β2 complex has been identified as the "high affinity" nicotine site. The homo-pentameric α7 receptor selectively binds the natural product, α-bungarotoxin, which has allowed its relatively facile localization and measurement. The α7 receptor is primarily expressed in the cortex, hippocampus and subcortical limbic regions and commonly occurs pre-synaptically. The localization of α7 nAChRs in areas involved with learning and memory has led to studies using both knockout mice and pharmacological manipulation. It is involved in sensory gating, memory, and neuronal plasticity. Alpha7 agonists have been shown to increase the release of neurotransmitters in rodents, including dopamine, serotonin, glutamate and GABA. Compounds which selectively bind to the α7 receptor, such as α7 agonists and partial agonists, have been shown to improve learning and memory functions in normal and aged animals, reverse scopolamine-induced memory deficits, reverse deficits in cognition induced by NMDA antagonists, reverse pharmacologically-induced gating deficits, e.g. amphetamine induced gating disruption, and to possess some anxiolytic properties. The α7 agonists of the present invention are expected to be useful in the treatment of schizophrenia and cognitive disorders associated with schizophrenia.

Alzheimer's disease is a progressive neurodegenerative disorder, resulting in the general loss of cognitive functions. The incidence increases with age, to the degree that 25-50% of all individuals over 85 are estimated to suffer from some degree of dementia. A diagnosis of Alzheimer's implies that the remaining life expectancy is reduced by half, compared to normal adults.

Clinical signs of Alzheimer's disease are progressive cognitive deterioration, decreased ability to perform the activities of daily living and neuropsychiatric symptoms or behavioral changes. In the advanced stages of the disease, deterioration of musculature and mobility may lead to inability to feed oneself, and eventually to the patient becoming bedridden. Language becomes severely disorganized, and then is lost altogether. Patients are not able to perform even simple tasks independently and require constant supervision. The cost of institutional care makes up nearly 70% of the cost of the disease. Therefore, therapies which increase cognitive function and delay institutionalization are greatly needed.

Alzheimer's disease has been shown in several studies to be accompanied by a reduction in nicotinic receptors in the cortex and hippocampus. Nicotine injections or nicotine skin patches have been reported to significantly improve attention, memory and learning in Alzheimer's disease patients. While there is a progressive loss of nicotinic receptors during the course of Alzheimer's disease, the α7 neurons are relatively spared, compared to the more abundant α4 receptors. Recently, the administration of selective nicotinic α7 agonists has been shown to increase cognitive functioning in Alzheimer's patients when dosed as long as 8 weeks. This clinical data is consistent with pre-clinical data showing α7 agonists and partial agonists improve learning and memory functions in normal and aged animals and reverse scopolamine-induced memory deficits. Thus, the compounds of the present invention may be useful in the treatment and prevention of Alzheimer's disease. The amyloid peptide Aβ42 has been shown to bind to the α7 nicotinic receptor (Wang et al., J. Biol. Chem., 2000, 275:5626-5632; J. Neurochem. 2000, 75:1155-1161). This association may facilitate the aggregation of Aβ42, believed to be important in the toxic effects of Aβ42, and may also cause disregulation of signaling through α7 nicotinic receptors. Deletion of the a7 receptor gene improves cognitive deficits and synaptic pathology in a mouse model of Alzheimer's disease (Dziewczapolski et al., J. Neuroscience, 2009, pp 8805-8815). The compounds of the present invention may disrupt the interaction of Aβ42 and α7 receptors. Treatment with α7 agonists and partial agonists may represent an approach for disease modification in Alzheimer's disease. Alpha7 receptors may also mediate inflammatory processes in neurodegenerative conditions, such as Alzheimer's disease (Conejero-Goldberg et al., Neurosci. and Biobehav. Rev., 2008, 32, pp 693-706). The α7 agonists and partial agonists of the present invention may be useful in reducing inflammation in neurodegenerative diseases and disorders, such as Alzheimer's disease.

The α7 receptor has also been shown to be involved in the reduction of inflammation via the vagus nerve. In addition, the α7 receptor is expressed in synoviocytes from RA and OA patients, and α7 agonists have been shown to inhibit the proinflammatory cascade that occurs in the rheumatoid joint (Waldberger et al., Arthritis and Rheumatism, Vol 58, pp 3439-3449). Thus, the compounds of the present invention may be useful in the treatment of inflammatory conditions, such as rheumatoid arthritis and osteoarthritis.

Nicotinic receptors containing the α7 subunit are present on mucosal mast cells known to be involved in gastrointestinal hypersensitivity (Kageyama-Yahara et al., Biochem and Biophys. Research Commun., 2008, v. 377, pp321-325). The α7 agonist GTS-21 inhibits the antigen-induced degranulation of mucosal mast cells, suggesting that α7 agonists may be useful in the treatment of hypersensitive bowel conditions, such as ulcerative colitis.

In a recent report (Marrero et al., JPET Fast Forward, September 28, 2009, DOI: 10.1124/jpet.109.154633), an α7 agonist was shown to decrease weight gain and food intake and reduce the elevated plasma levels of triglycerides, glucose, glycated hemoglobin and TNFa in a mouse model of type II diabetes (db/db mice which are deficit in leptin receptors). The α7 agonists and partial agonists of the present invention may be useful in the treatment of diabetes.

The following references provide general reviews of the nicotinic receptor system and α7 receptors and ligands: Picciotto and Zoli, J. Neurobio. (2002) 53:641-655; Brening, et al, Ann. Reports in Med. Chem. (2005) 40:3-16; Dani and Bertrand, Ann. Rev. Pharm. Tox. (2007) 47:699-729; Olincy and Stevens, Biochem. Pharmacol. (2007) 74:1192-1201; Broad, et al, Drugs Future (2007) 32 (2):161-70; de Jonge and Ulloa, Brit. J. Pharmacol. (2007) 151:915-929; Romanelli, et al, ChemMedChem (2007) 2(6):746-767; Lightfoot et al., Progress in Medicinal Chemistry (2008), v 46, pp131-171; Concotta et al., Current Opinion in Investigational Drugs (2008), v 9, pp47-56; Leiser et al., Pharmacol. and Therapeutics (2009), doi:10:1016/j.pharmthera.2009.03.009).

Ligands for the nicotinic α7 receptor have been disclosed in the references above, and also in US patent application publication U.S. 2007004715, WO 2008/000469, WO 2003/092580, WO 2004/000,469, EP 337,547, EP 452,101, and C. Swain, et al., J. Med. Chem., (1992) 35:1019-1031.

The invention provides technical advantages, for example, the compounds are novel and are ligands for the nicotinic α7 receptor and may be useful for the treatment of various disorders of the central nervous system, especially affective and neurodegenerative disorders. Additionally, the compounds provide advantages for pharmaceutical uses, for example, with regard to one or more of their mechanism of action, binding, inhibition efficacy, target selectivity, solubility, safety profiles, or bioavailability.

### DESCRIPTION OF THE INVENTION

The invention encompasses a compound selected from the group consisting of 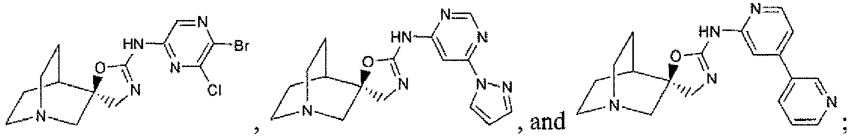 or a pharmaceutically acceptable salt thereof, and compositions comprising said compounds or salts, and said compounds or salts for use in a method of treatment. The compounds may be useful for the treatment of various disorders of the central nervous system:
The compounds of the invention belong to the group of compounds of formula I, or a stereoisomer thereof, 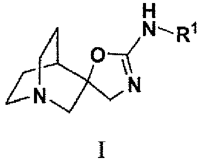 wherein:
   R¹ is selected from the group consisting of isoxazolyl, pyrazolyl, oxazolyl, thiazolyl, imidazolyl, oxadiazolyl, thiadiazolyl, triazolyl, pyridinyl, pyrazinyl, pyridazinyl, pyrimidinyl, triazinyl, quinolinyl, isoquinolinyl, tetrahydroisoquinolinyl, quinoxalinyl, quinazolinyl, naphthyridinyl, indazolyl, indolyl, 2-indolonyl, benzisoxazolyl, benzoisothiazolyl, benzoxazolyl, benzothiazolyl, benzimidazolyl, furopyridinyl, thienopyridinyl, thienopyrimidinyl, isothiazolopyridinyl, thiazolopyridinyl, thiazolopyridinonyl, thiazolopyrazinyl, thiazolopyrimidinyl, triazolopyridinyl, triazolopyrazinyl, pyrrolotriazinyl, 5,6-dihydrobenzo[h]quinazolinyl, 5H-chromeno[4,3-d]pyrimidinyl, 6,7-dihydro-5H-cyclopenta[d]pyrimidinyl, 5,6,7,8-tetrahydroquinazolinyl, 7,8-dihydroquinazolin-5(6H)-onyl, and tetrahydrobenzothiazolyl, and is substituted with 0-3 substituents independently selected from the group consisting of C₁₋₄alkyl, C₃₋₇cycloalkyl, C₁₋₄haloalkyl, C₁₋₄alkoxy, C₁₋₄haloalkoxy, C₃₋₇cycloalkoxy, C₁₋₄alkylthio, phenoxy, benzyloxy, halo, hydroxy, cyano, nitro, C₁₋₄alkylsulfonyl, NR²R³, pyrrolidinonyl, methylenedioxy, furyl, thienyl, pyrazolyl, imidazolyl, thiazolyl, triazolyl, pyrazinyl, pyrimidinyl, naphthyl, C₁₋₄alkylamido, CONR²R³, pyridyl, phenyl, and benzyl, and where imidazolyl, pyridyl, phenyl and benzyl are substituted with 0-2 substituents independently selected from the group consisting of halo, C₁₋₄alkyl, C₁₋₄alkoxy, C₁₋₄haloalkyl, C₁₋₄haloalkoxy, and NR²R³;
   R² is hydrogen, C₁₋₄alkyl, C₁₋₄hydroxyalkyl, or C₁₋₄aminoalkyl;
   R³ is hydrogen, C₁₋₄alkyl, C₁₋₄hydroxyalkyl, or C₁₋₄aminoalkyl;
   or R₂ and R₃ taken together with the nitrogen atom to which they are attached is azetidinyl, pyrrolidinyl, piperidinyl, piperazinyl, N-(C₁₋₄alkyl)piperazinyl, morpholinyl, or homopiperidinyl;
      or a pharmaceutically acceptable salt thereof.

According to another aspect, the compounds of the invention belong to the group of stereoisomers of formula I according to formula Ia.

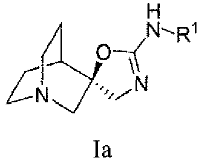

Unless specified otherwise, these terms have the following meanings. "Alkyl" means a straight or branched alkyl group composed of 1 to 4 carbons. "Alkenyl" means a straight or branched alkyl group composed of 2 to 4 carbons with at least one double bond. "Alkynyl" means a straight or branched alkyl group composed of 2 to 4 carbons with at least one triple bond. "Cycloalkyl" means a monocyclic ring system composed of 3 to 7 carbons. "Haloalkyl" and "haloalkoxy" include all halogenated isomers from monohalo to perhalo. Terms with a hydrocarbon moiety (e.g. alkoxy) include straight and branched isomers for the hydrocarbon portion. Parenthetic and multiparenthetic terms are intended to clarify bonding relationships to those skilled in the art. For example, a term such as ((R)alkyl) means an alkyl substituent further substituted with the substituent R.

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Some of the compounds of the invention exist in stereoisomeric forms. The invention includes all stereoisomeric forms of the compounds including enantiomers and diastereromers. Methods of making and separating stereoisomers are known in the art.

The invention includes all tautomeric forms of the compounds. An example of a tautomeric pair is shown below.

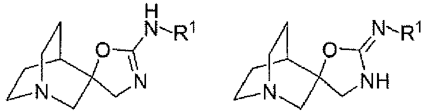

The invention is intended to include all isotopes of atoms occurring in the present compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties.

### Synthetic Methods

The compounds may be made by methods known in the art including those described below and including variations within the skill of the art. Some reagents and intermediates are known in the art. Other reagents and intermediates can be made by methods known in the art using readily available materials. The variables (e.g. numbered "R" substituents) used to describe the synthesis of the compounds are intended only to illustrate how to make the compounds and are not to be confused with variables used in the claims or in other sections of the specification. The following methods are for illustrative purposes and are not intended to limit the scope of the invention.

Some of the compounds may be prepared using the reactions and techniques described in this section. The reactions are performed in solvents appropriate to the reagents and materials employed and are suitable for the transformations being effected. It is understood by one skilled in the art of organic synthesis that the functionality present on various portions of the molecule must be compatible with the reagents and reactions proposed. Such restrictions to the substituents which are compatible with the reaction conditions will be readily apparent to one skilled in the art and alternate methods must then be used.

Abbreviations used in the schemes generally follow conventions used in the art. Chemical abbreviations used in the specification and examples are defined as follows: "NaHMDS" for sodium bis(trimethylsilyl)amide; "DMF" for N,N-dimethylformamide; "MeOH" for methanol; "NBS" for N-bromosuccinimide; "Ar" for aryl; "TFA" for trifluoroacetic acid; "LAH" for lithium aluminum hydride; "BOC", "DMSO" for dimethylsulfoxide; "h" for hours; "rt" for room temperature or retention time (context will dictate); "min" for minutes; "EtOAc" for ethyl acetate; "THF" for tetrahydrofuran; "EDTA" for ethylenediaminetetraacetic acid; "Et₂O" for diethyl ether; "DMAP" for 4-dimethylaminopyridine; "DCE" for 1,2-dichloroethane; "ACN" for acetonitrile; "DME" for 1,2-dimethoxyethane; "HOBt" for 1-hydroxybenzotriazole hydrate; "DIEA" for diisopropylethylamine, "Nf" for CF₃(CF₂)₂SO₂-; and "TMOF" for trimethylorthoformate.

Abbreviations as used herein, are defined as follows: "1 x" for once, "2 x" for twice, "3 x" for thrice, "°C" for degrees Celsius, "eq" for equivalent or equivalents, "g" for gram or grams, "mg" for milligram or milligrams, "L" for liter or liters, "mL" for milliliter or milliliters, "µL" for microliter or microliters, "N" for normal, "M" for molar, "mmol" for millimole or millimoles, "min" for minute or minutes, "h" for hour or hours, "rt" for room temperature, "RT" for retention time, "atm" for atmosphere, "psi" for pounds per square inch, "conc." for concentrate, "sat" or "sat'd " for saturated, "MW" for molecular weight, "mp" for melting point, "ee" for enantiomeric excess, "MS" or "Mass Spec" for mass spectrometry, "ESI" for electrospray ionization mass spectroscopy, "HR" for high resolution, "HRMS" for high resolution mass spectrometry , "LCMS" for liquid chromatography mass spectrometry, "HPLC" for high pressure liquid chromatography, "RP HPLC" for reverse phase HPLC, "TLC" or "tlc" for thin layer chromatography, "NMR" for nuclear magnetic resonance spectroscopy, "¹H" for proton, "δ" for delta, "s" for singlet, "d" for doublet, "t" for triplet, "q" for quartet, "m" for multiplet, "br" for broad, "Hz" for hertz, and "α", "β", "R", "S", "E", and "Z" are stereochemical designations familiar to one skilled in the art.

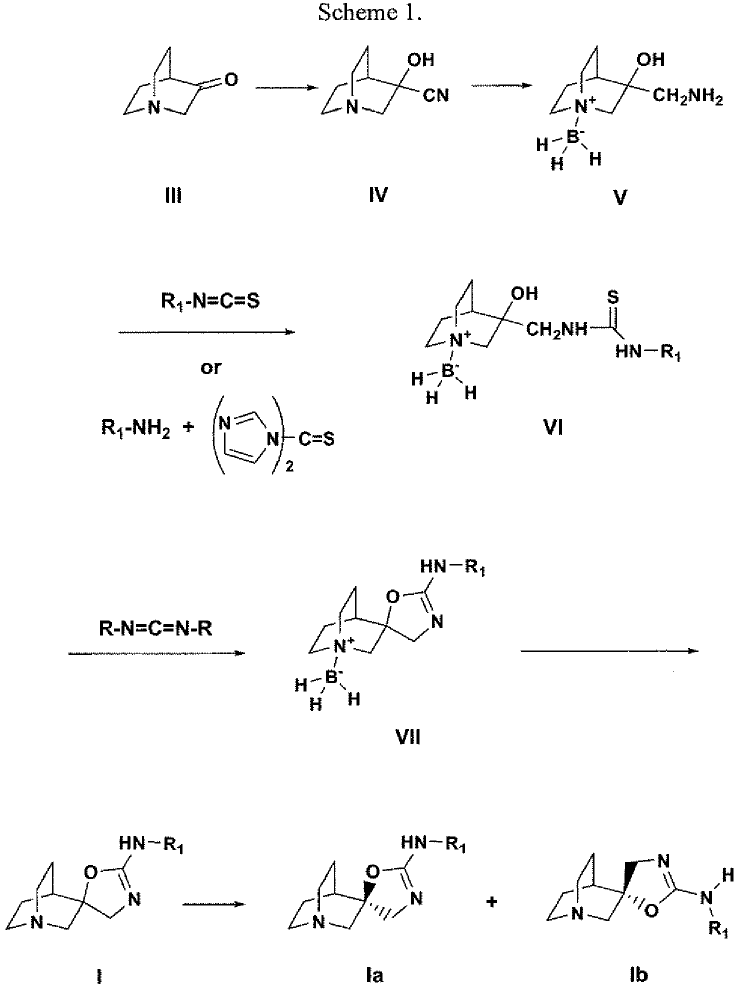

Compounds of Formula I are prepared as illustrated in Reaction Scheme 1. The ketone of Formula III (3-quinuclidone) is known, is commercially available, or may be prepared by methods known to those skilled in the art. The ketone can be converted to the corresponding cyanohydrin of Formula IV by reaction with sodium or potassium cyanide plus an acid. The compound of Formula IV can be reduced to the corresponding amino-methyl compound (borane complex) of Formula V by reaction with borane/tetrahydrofuran complex.

The compound of Formula V can be reacted with heteroaryl isothiocyanates directly in an inert solvent to give the thioureas of Formula VI. Alternatively, the heteroarylamine can be reacted with thiocarbonyl-diimidazole to give an activated species which can be used without isolation to convert the compound of Formula V to the compound of Formula VI. The heteroarylamine may be prepared by methods known to those skilled in the art.

The thiourea of Formula VI can be cyclized using, for example, di-isopropyl carbodiimide to give the oxazoline of Formula VII which may be deprotected via treatment with acid to give the racemic final product of the compound of Formula I. The compound of Formula I may be resolved into pure enantiomer compounds of Formula Ia and Formula Ib by means known in the art, for example, via chiral chromatography.

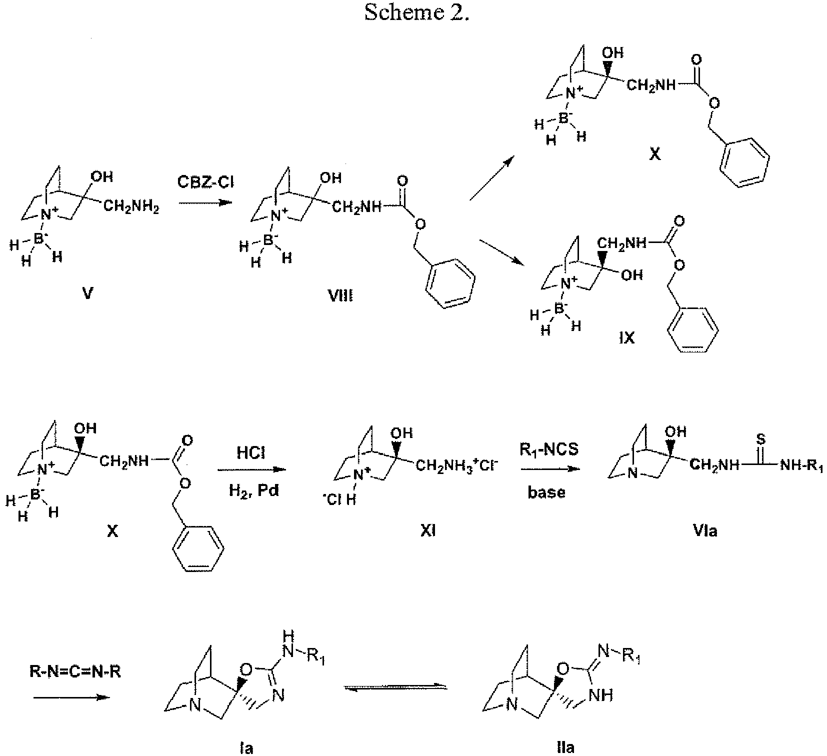

Alternatively, the free amino group of the quinuclidine of Formula V can be blocked with, for example, carbobenzyloxy-chloride ("CBZ-Cl") to give the compound of Formula VIII, as illustrated in Reaction Scheme 2.

The racemic compound of Formula VIII can be resolved into its enantiomers, Formula IX and Formula X by, for example, chiral chromatography. Either the compound of Formula IX or Formula X, and preferably the compound of Formula X, can then be carried on as shown in Reaction Scheme 2.

The borane group in the compound of Formula X can be removed, for example, by treatment with dilute hydrochloric acid, and the carbobenzyloxy group can be removed, for example, by catalytic hydrogenation to give the chiral quinuclidine amine of Formula XI. Similarly to Reaction Scheme 1, the amine salt of Formula XI can be reacted with isothiocyanates to give the thiourea of Formula VIa, which can then be reacted with dialkyl carbodiimides or mixed thioureas (as from reaction with thiocarbonyl diimidazole) to give the chiral oxazoline quinuclidine compounds of Formula Ia, and its tautomer, Formula IIa.

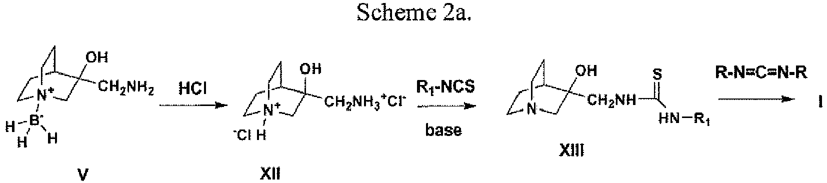

Alternatively, the borane group of V may be removed with hydrochloric acid to give dihydrochloride salt XII, which can be reacted in the presence of base with an isothiocyanate to give intermediate thiourea XIII, which can then be cyclized as in Reaction Schemes 1-2 to give I. XII may also be prepared by other methods, as referenced in US Patent #5,137,895 (8/11/1992).

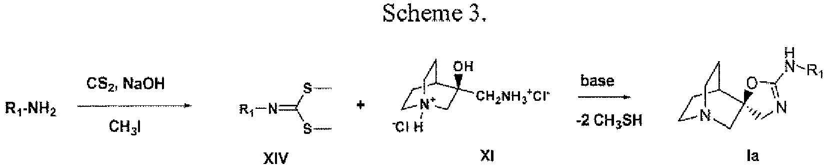

Additionally, the (hetero)aromatic amines may be reacted with carbon disulfide, sodium hydroxide, and methyl iodide to give intermediate dimethyl carbonimidodithioates XIV. These are reacted with dihydrochloride XI in the presence of base to eliminate two moles of methanethiol and generate desired products Ia directly.

### Biological methods

*I)* α7 *Nicotinic Acetycholine Receptor Binding.* Membranes were prepared for binding using HEK293 cells stably expressing the rat α7 nicotinic acetycholine receptor (rat α7 nAChR). Cells were homogenized at 4°C in hypotonic lysis buffer consisting of 10 mM Tris (pH 7.4), 5 mM EDTA and protease inhibitors and centrifuged at 32000 xg for 20 minutes. The pellet was washed once in membrane wash buffer consisting of 50 mM Tris (pH 7.4), 1 mM EDTA and protease inhibitors and centrifuged at 32000 xg for 20 minutes. This pellet was then resuspended in assay buffer consisting 50 mM KH₂PO₄ (pH 7.4 at 25°C), 1 mM EDTA, 0.005 % Triton-X 100 and 0.1% (v/v) Sigma Protease Inhibitor Cocktail. Aliquots were then frozen in dry ice/ethanol and kept at -80°C until the day of the assay.
*II) A Ca*^{*2*+} *-Sensitive, Fluorescence-Based Assay* α*-7 for Nicotinic Acetylcholine Receptor Channel Function In Mammalian Cells ("FLIPR").* Summary: Lead compounds are evaluated for agonist activity at α-7, α3β4, α4αβ2, and α1β1δ1ε sub-types of nicotinic ACh receptor ion channels expressed in mammalian HEK 293 cells. Agonist potency and efficacy values are determined from kinetic fluorescence Ca²⁺ influx measurements made using a 384 well FLIPR (Fluorescence Image Plate Reader). The utility of fluorescent indicators for measuring changes in intracellular divalent cation concentrations, particularly Ca²⁺, for drug discovery endeavors is well documented (Rudiger, R., et al., Nature Reviews, 2003, 4:579-586; Gonzalez J.E., et al., Receptors and Channels, 2002, 8:283-295). In this assay, channel expressing HEK cell lines seeded in 384 well assay plates are loaded with a membrane permeant fluorescent Ca²⁺ indicator dye, whose 510 nm green emission signal increases in response to elevation of intracellular Ca²⁺ concentration. The basal fluorescence from the cells is monitored in real time, followed by the acute addition of test compounds. If the compound is an agonist at any of the non-selective cation channels, the latter open and allow the movement of extracellular Ca²⁺ ions into the cell cytoplasm, where they bind to the Ca²⁺ indicator dye, and produce an increase in fluorescence output signal, which is detected by a cooled CCD imaging camera.
   Materials and Methods: Reagents: The acetomethoxy (AM) ester of the Ca²⁺ indicator dye Fluo-4 was obtained from InVitrogen, (Carlsbad, CA). Acetylcholine and all buffer constituents were purchased from Sigma Chemical Company, St. Louis, MO. G418 and Minimal Essential Medium were purchased from InVitrogen Life Technologies, Carlsbad, CA. Fetal bovine serum was purchased from (InVitrogen, Carlsbad, CA).
   Cell Culture: HEK-293 cells were grown in Minimal Essential Medium containing 10% (v/v) fetal bovine serum at 37° C in a 5% CO₂ incubator. HEK-293 cells stably expressing the ion channels were grown in the same medium with the addition of 500 µg/ml G418.
   Ca²⁺ flux assays of Ca ²⁺ channels expressed in HEK-293 cells: HEK-293 cells expressing the ion channels of interest were plated in 384 well, black-walled, clear-bottomed, poly-D-lysine coated plates at a density of ~20,000 cells/well in 20 µl of Minimal Essential Medium containing 10% (v/v) fetal bovine serum and incubated for 2 days at 29° C in a 5% CO₂ incubator. Prior to assay, cells were loaded with the Fluo-4 AM ester. Cell loading was accomplished by removing the culture medium and replacing it with 30 µl/well of the AM ester of the dye (5 µM) mixed with Hanks Balanced Salt Solution (#14175-095) containing 20mM HEPES, 2.5mM probenecid,0.5mM CaCl₂, I mM MgCl2 and 10µM atropine. Dye loading was allowed to proceed for 90 minutes at room temperature at which time the dye loading solution was removed and replaced with 40 µl/well of Hanks buffer. Cells loaded with dye were loaded onto a FLIPR384 (Molecular Devices, Sunnyvale, CA). Fluo-4 dye was excited using the 488 nm line of an argon laser. Emission was filtered using a 540 +/- 30 nm bandpass filter. For evaluation of the effects of test compounds using the Ca²⁺ flux assay, compounds to be tested were provided in assay ready plates. For nicotinic receptor ion channel expressing cells, the assay was initiated by the addition of 20 µl/well of Hanks buffer containing test compounds. For all assays, data were collected at 1 Hz for 10 seconds (baseline), at which time the compound containing stimulus buffers are added, and further measurements collected at 0.33Hz for 3 min.
   Data Analysis: The statistical robustness of the nicotinic receptor Ca²⁺ flux assays is determined from blanks and totals wells. The totals wells define maximal channel activation for each compound test plate (Maximum efficacious dose of acetylcholine), and the blanks wells which contain matched DMSO only, define zero channel activation. The raw fluorescence units data files generated on the FLIPR plate reader are automatically exported and processed by in-house data analysis tools. The reduced percent activation data for each concentration of test compound are fit using MathIQ fitting engine (ID Business Solutions Limited, Surrey, UK). Data were analyzed by fitting maximum amplitudes of change in fluorescence, for Ca²⁺ flux for a given condition of test compound. Potencies (EC₅₀ values) of compounds are calculated from the average of three assay wells from a twenty point CRC. Test compound efficacy values (Ymax values) are expressed relative to a maximal response to acetylcholine in the total wells.
*III) Fos quantification assay:* Male Wistar rats are treated with drug (1-10 mg/kg) or vehicle (2 ml/kg, sc). Two hours after treatments, the rats are rapidly decapitated and discrete brain regions of interest are isolated on ice and weighed and flash frozen with liquid nitrogen and stored at -80 deg. C. Further processing of the brain tissue for nuclear extracts as well as for Fos quantification are in accordance with the protocol prescribed by a commercially available ELISA-based chemiluminiscence detection kit (catalog #89860, EZ-detect c-Fos Trans kit, Pierce Biotechnology Inc., Rockford, IL).
*IV) MK-801 Disrupted Set-Shift Assay in rats:* This assay uses a modification of the protocol described by Stefani et al. (Behavioral Neuroscience, 2003, 117: 728-737). Test compounds are assessed for their ability to reverse an MK-801-induced performance deficit (0.03 mg/kg, i.p., single dose) in this assay.

The activity of specific compounds described herein and tested in the above assay (II) is provided in Table 1.

**TABLE 1**

| | | | |
|---|---|---|---|
| 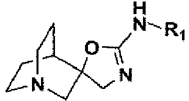 | | | |

| Example Number | R₁ | FLIPR α7 (EC₅₀, nM) | FLIPR α7 activity^{a} rating (EC₅₀, nM) |
|---|---|---|---|
| 1 | 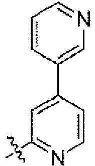 | | +++ |
| 2 | 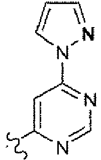 | NT | |

| | | | |
|---|---|---|---|
| ^{a} Activity based on EC₅₀ nM values: +++ = < 100 nM; ++ = 100 - 1000 nM; + = 1000 - 100000 nM; ^{b}NT = Not tested. | | | |

### Pharmaceutical Compositions and Methods of Treatment

Compounds of formula I bind to alpha 7 and can be useful in treating affective disorders and neurodegenerative disorders. Therefore, another aspect is a composition comprising a compound of formula I, or a pharmaceutically acceptable salt thereof, and a pharmaceutically acceptable carrier.

Another aspect is the use of a compound of formula I in the manufacture of a medicament for the treatment of affective disorders or neurodegenerative disorders.

Another aspect is the use of a compound of formula I in the manufacture of a medicament for the treatment of schizophrenia or Alzheimer's Disease.

Another aspect is a compound of formula (I) for use in a method of treating affective disorders or neurodegenerative disorders.

Another aspect is a compound of formula (I) for use in a method of treating schizophrenia or Alzheimer's Disease.

Another aspect is a compound of formula (I) for use in a method of treating schizophrenia.

Another aspect is a method of treating Alzheimer's Disease comprising comprising administering to a patient a therapeutically effective amount of a compound of formula I.

Another aspect is a compound of formula (I) for use in a method of treating cognitive disorders.

Another aspect is a compound of formula (I) for use in a method of treating rheumatoid arthritis.

Another aspect is a compound of formula (I) for use in a method of treating osteoarthritis.

Another aspect is a compound of formula (I) for use in a method of treating ulcerative colitis.

Another aspect is a compound of formula (I) for use in a method of treating Crohn's Disease.

Another aspect is a compound of formula (I) for use in a method of treating diabetes.

"Patient" means a person suitable for therapy as understood by practitioners in the field of affective disorders and neurodegenerative disorders.

"Treatment," "therapy," and related terms are used as understood by practitioners in the field of affective disorders and neurodegenerative disorders.

The compounds of this invention are generally given as pharmaceutical compositions comprised of a therapeutically effective amount of a compound or its pharmaceutically acceptable salt and a pharmaceutically acceptable carrier and may contain conventional excipients. Pharmaceutically acceptable carriers are those conventionally known carriers having acceptable safety profiles. Compositions encompass all common solid and liquid forms including for example capsules, tablets, losenges, and powders as well as liquid suspensions, syrups, elixers, and solutions. Compositions are made using common formulation techniques, and conventional excipients (such as binding and wetting agents) and vehicles (such as water and alcohols) are generally used for compositions. See, for example, Remington's Pharmaceutical Sciences, Mack Publishing Company, Easton, PA, 17th edition, 1985.

Solid compositions are normally formulated in dosage units and compositions providing from about 1 to 1000 mg of the active ingredient per dose are preferred. Some examples of dosages are 1 mg, 10 mg, 100 mg, 250 mg, 500 mg, and 1000 mg. Generally, other agents will be present in a unit range similar to agents of that class used clinically. Typically, this is 0.25-1000 mg/unit.

Liquid compositions are usually in dosage unit ranges. Generally, the liquid composition will be in a unit dosage range of 1-100 mg/mL. Some examples of dosages are I mg/mL, 10 mg/mL, 25 mg/mL, 50 mg/mL, and 100 mg/mL. Generally, other agents will be present in a unit range similar to agents of that class used clinically. Typically, this is 1-100 mg/mL.

Generally, the dosing regimen will be similar to other agents used clinically. Typically, the daily dose will be 1-100 mg/kg body weight daily. Generally, more compound is required orally and less parenterally. The specific dosing regime, however, will be determined by a physician using sound medical judgement.

### DESCRIPTION OF SPECIFIC EMBODIMENTS

¹H-NMR spectra were run on a Bruker 500, 400, or 300 MHz instrument and chemical shifts were reported in ppm (δ) with reference to tetramethylsilane (δ=0.0). All evaporations were carried out under reduced pressure. Unless otherwise stated, LC/MS analyses were carried out on a Shimadzu instrument using a Phenomenex-Luna 4.6x50mm S 10 reverse phase column employing a flow rate of 4 mL/min using a 0.1% TFA in methanol/water gradient [0-100% in 3 min, with 4 min run time] and a UV detector set at 220 nm or Gemini C18 4.6x50mm 5u reverse phase column employing a flow rate of 5 mL/min using a 10 mM ammonium acetate acetonitrile/water gradient [5-95% in 3 min, with 4 min run time] and a UV detector set at 220 nm (negative-ion mass spectrometry). Unless otherwise stated, purification could be done by preparative C-18 column employing gradients of methanol-water containing 0.1% of trifluoroacetic acid (TFA), and using a Shimadzu High Performance Liquid Preparative Chromatographic System employing an XTERRA 30x100 mm S5 column at 40 mL/min flow rate with a 12 min gradient. The present invention is directed only to the specific examples encompassed by the current set of claims. All other structures are reference embodiments.

### REFERENCE EXAMPLE 1

### (R)-N-(6-Methoxypyrimidin-4-yl)-4H-1'-azaspiro[oxazole-5,3'-bicyclo[2.2.2]octan]-2-amine

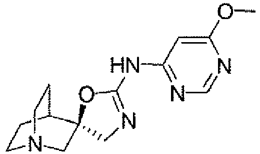

### Step A: 4-Isothiocyanato-6-methoxypyrimidine

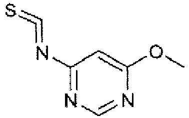

To a bright orange solution of 1,1'-thiocarbonyldipyridin-2(1H)-one (1.86 g, 7.99 mmol) in dichloromethane at room temperature was added 6-methoxypyrimidin-4-amine (1 g, 8 mmol). The orange solution was stirred at room temperature for 18 hours. The LC/MS showed the desired product as one of the major peaks. The deep orange solution was concentrated and the remaining residue was filtered. The filtrate was purified by silica gel chromatography (10-50% ethyl acetate/hexanes) to afford 4-isothiocyanato-6-methoxypyrimidine (0.72 g, 4.3 mmol, 54 % yield) as a yellow oil. ¹H NMR (400 MHz, DMSO-*d*₆) δ ppm 8.49 (1 H, d, J=5.79 Hz), 6.95 (1 H, d, J=5.79 Hz), 3.92 (3 H, s). MS (LC/MS) R.T. = 3.15; [M+H]⁺ = 168.1.

### Step B: (R)-N-(6-Methoxypyrimidin-4-yl)-4H-1-azaspiro[oxazole-5,3'-bicyclo[2.2.2]octan]-2-amine

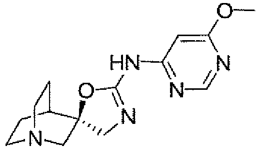

To (S)-3-(aminomethyl)quinuclidin-3-ol dihydrochloride (from Step B of Example 21) (0.34 g, 1.49 mmol) in N,N-dimethylformamide (15 mL) was added Cs₂CO₃ (1.22 g, 3.74 mmol) and 4-isothiocyanato-6-methoxypyrimidine (0.25 g, 1.5 mmol). The suspension was stirred at room temperature for 30 minutes. N,N'-diisopropylcarbodiimide (0.7 mL, 4.5 mmol) was then added and the mixture was stirred at room temperature for 18 hours. The mixture was concentrated and purified by silica gel chromatography (5-15 % [9:1 methanol:ammonium hydroxide]/chloroform) to afford (R)-N-(6-methoxypyrimidin-4-yl)-4H-1'-azaspiro[oxazole-5,3'-bicyclo[2.2.2]octan]-2-amine (0.21 g, 0.72 mmol, 48.2% yield) as a white solid. M.P. 186-8 °C. ¹H NMR (400 MHz, MeOD) δ ppm 8.40 (1 H, s), 6.17 (1 H, br. s.), 3.92 - 4.04 (1 H, m), 3.89 (3 H, s), 3.68 (1 H, d, *J*=10.32 Hz), 3.12 - 3.23 (1 H, m), 2.98 - 3.12 (1 H, m), 2.67 - 2.97 (4 H, m), 2.11 (2 H, br. s.), 1.48 - 1.82 (3 H, m). MS (LC/MS) R.T. = 0.82; [M+H]⁺ = 290.3.

### REFERENCE EXAMPLE 2

### (R)-N-(5-Phenylpyridin-3-yl)-4H-1'-azaspiro[oxazole-5,3'-bicyclo[2.2.2]octan]-2-amine

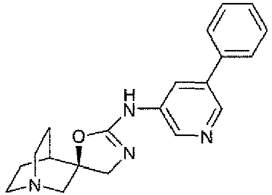

### Step A: 5-Phenylpyridin-3-amine

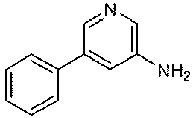

A mixture of 5-bromopyridin-3-amine (248 mg, 1.43 mmol), Pd(PPh₃)₄ (50.4 mg, 0.04 mmol), toluene (3 mL), sodium carbonate (2 M, 3 mL, 6 mmol), and phenylboronic acid (195 mg, 1.60 mmol) dissolved in ethanol (3 mL) was heated for 4 hours in an oil bath at 90 °C and allowed to cool to room temperature for 16 hours. The reaction mixture was transferred to a separatory funnel and partitioned between ethyl acetate and water. The aqueous phase was washed once more with ethyl acetate, and the combined organic phases were washed with brine and dried over magnesium sulfate, filtered and concentrated *in vacuo.* The residue was purified by column chromatography (80% ethyl acetate/hexanes) to afford 5-phenylpyridin-3-amine (31.9 mg, 0.19 mmol, 13 % yield) as a white solid. ¹H NMR (500 MHz, *CDCl₃*) δ ppm 8.17 - 8.42 (m, 1 H), 8.02 - 8.20 (m, 1 H), 7.32 - 7.62 (m, 4 H), 7.25 (s, 1 H), 7.06 - 7.20 (m, 1 H). MS (LC/MS) R.T. = 0.91; [M+H]⁺ = 171.09.

### Step B: (R)-N-(5-Phenylpyridin-3-yl)-4H-1'-azaspiro[axazole-5,3'-bicyclo[2.2.2]octan]-2-amine

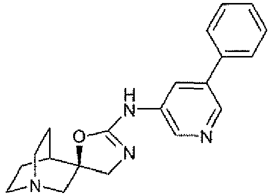

(R)-N-(5-Phenylpyridin-3-yl)-4H-1'-azaspiro[oxazole-5,3'-bicyclo[2.2.2]octan]-2-amine was prepared by following the general procedures of **Reference Example 1, Steps A-B** using 5-phenylpyridin-3-amine (**from Step A above**) as the starting material. ¹H NMR (500 MHz, *MeOD-d₄*) δ ppm 8.40 (br. s., 2 H), 7.66 (d, *J*=7.32 Hz, 2 H), 7.46 - 7.56 (m, 3 H), 7.43 (d, *J*=7.32 Hz, 1 H), 3.79 - 4.02 (m, 1 H), 3.51 - 3.68 (m, 1 H), 3.22 (d, *J*=14.95 Hz, 1 H), 3.02 - 3.15 (m, 1 H), 2.72 - 2.99 (m, 3 H), 2.14 (br. s., 2 H), 1.76 (dd, *J*=9.31, 4.12 Hz, 3 H), 1.12 - 1.35 (m, 1 H). MS (LC/MS)
R.T. = 0.90; [M+H]⁺ = 335.17.

### EXAMPLE 1

### (R)-N-(3,4'-Bipyridin-2'-yl)-4H-1'-azaspiro[oxazole-5,3'-bicyclo[2.2.2]octan]-2-amine

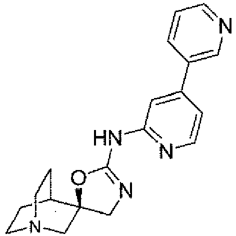

(R)-N-(3,4'-Bipyridin-2'-yl)-4H-1'-azaspiro[oxazole-5,3'-bicyclo[2.2.2]octan]-2-amine was prepared from 4-bromopyridin-2-amine, pyridin-3-ylboronic acid, and 1,1'-bis(diphenylphosphino)ferrocene-palladium(II)dichloride dichloromethane complex by following the general procedures of Reference **Example 2, Steps A-B.** ¹H NMR (500 MHz, *DMSO-d*₆) δ ppm 8.95 (br. s., 2 H), 8.66 (d, *J*=4.6 Hz, 1 H), 8.31 (d, *J*=5.5 Hz, 1 H), 8.14 (br. s., 1 H), 7.54 (d, *J*=7.6 Hz, 1 H), 7.26 (br. s., 2 H), 3.85 (br. s., 1 H), 3.58 (s, 1 H), 3.00 (br. s., 2 H), 2.67 (br. s., 4 H), 1.90 (br. s., 2 H), 1.59 (br. s., 2 H), 1.45 (br. s., 1 H). MS (LC/MS) R.T. = 0.12; [M+H]⁺ = 336.18.

### EXAMPLE 2

### (R)-N-(6-(1H-Pyrazol-1-yl)pyrimidin-4-yl)-4H-1'-azaspiro[oxazole-5,3'-bicyclo[2.2.2]octan]-2-amine

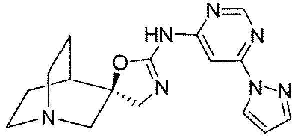

### Step A: 4-Chloro-6-(1H-pyrazol-1-yl)pyrimidine

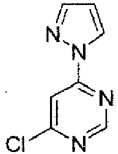

A mixture of 4,6-dichloropyrimidine (5.96 g, 40 mmol), 1H-pyrazole (2.72 g, 40.0 mmol), and cesium carbonate (13.03 g, 40.0 mmol) was stirred in DMF (25 mL) at room temperature for 18 h. The reaction was diluted into 100 mL water and extracted with EtOAc. The combined organic layers were concentrated and purified by flash chromatography on a 240 g silica gel cartridge with 0 to 20% ethyl acetate in hexane to yield 4-chloro-6-(1H-pyrazol-1-yl)pyrimidine (4.80 g, 66 % yield). 1H NMR (400 MHz, CHLOROFORM-d) δ ppm 8.77 (1 H, d, J=0.76 Hz), 8.55 (1 H, d, J=2.77 Hz), 7.96 (1 H, d, J=1.01 Hz), 7.80 (1 H, d, J=1.01 Hz), 6.51 (1 H, dd, J=2.64, 1.64 Hz).
LCMS: RT = 0.86 min, MH+ = 181.1.

### Step B: 6-(1H-Pyrazol-1-yl)pyrimidin-4-amine

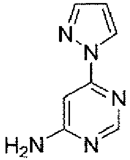

A solution of 4-chloro-6-(1H-pyrazol-1-yl)pyrimidine (1.31 g, 7.25 mmol) in 2 M ammonia (20 mL, 40.0 mmol) / isopropanol was heated at 80 °C in a sealed vial for 24 h. The reaction was stored at room temperature for 4 days. The solid precipitate was filtered and dried to give 6-(1H-pyra.zol-1-yl)pyrimidin-4-amine (1.15 g, 98 % yield). 1H NMR (400 MHz, DMSO-d6) δ ppm 8.53 (1 H, dd, J=2.52, 0.50 Hz), 8.30 (1 H, d, J=0.76 Hz), 7.82 (1 H, d, J=1.01 Hz), 7.19 (2 H, br. s.), 6.88 (1 H, d, J=1.01 Hz), 6.55 (1 H, dd, J=2.64, 1.64 Hz). LCMS: RT = 0.52 min, MH+ = 162.1.

### Step C: Dimethyl 6-(1H-pyrazol-1-yl)pyrimidin-4-ylcarbonimidodithioate

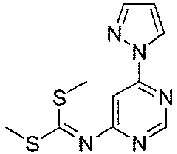

To a solution of 6-(1H-pyrazol-1-yl)pyrimidin-4-amine (1.0 g, 6.20 mmol) in DMF (18 mL) was added dropwise 10 M NaOH (1.24 mL, 12.4 mmol), carbon disulfide (0.933 mL, 15.5 mmol), NaOH (1.24 mL, 12.4 mmol), and iodomethane (0.966 mL, 15.5 mmol) at 15 min intervals. Stirring was continued overnight and the mixture was poured into water. The tan precipitate was filtered, washed with water, and dried to yield dimethyl 6-(1H-pyrazol-1-yl)pyrimidin-4-ylcarbonimidodithioate (136 mg, 8 % yield).
1H NMR (400 MHz, MeOD) δ ppm 8.74 (1 H, d, J=1.01 Hz), 8.64 (1 H, d, J=2.52 Hz), 7.82 (1 H, d, J=1.26 Hz), 7.40 (1 H, d, J=1.01 Hz), 6.57 (1 H, dd, J=2.64, 1.64 Hz), 2.58 (6 H, s). LCMS: RT = 0.96 min, MH+ = 266.1.

### Step D: (R)-N-(6-(1H-Pyrazol-1-yl)pyrimidin-4-yl)-4H-1'-azaspiro[oxazole-5,3'-bicyclo[2.2.2]octan]-2-amine

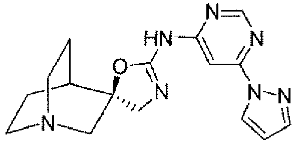

A suspension of (S)-3-(aminomethyl)quinuclidin-3-ol dihydrochloride (135 mg, 0.59 mmol), dimethyl 6-(1H-pyrazol-1-yl)pyrimidin-4-ylcarbonimidodithioate (130 mg, 0.49 mmol), and cesium carbonate (399 mg, 1.23 mmol) was stirred in DMF (1.2 mL) at 75 °C for 2 h. The reaction was concentrated and purified by flash chromatography on a 40 g silica gel cartridge with a pre-run of 1% [95:5 MeOH/NH4OH] in EtOAc, then isocratic 1% [95:5 MeOH/NH4OH] in CHCl3 to yield (R)-N-(6-(1H-pyrazol-1-yl)pyrimidin-4-yl)-4H-1'-azaspiro[oxazole-5,3'-bicyclo[2.2.2]octan]-2-amine (105 mg, 66% yield). 1H NMR (400 MHz, CHLOROFORM-d) δ ppm 9.46 (1 H, br. s.), 8.58 (1 H, d, J=1.01 Hz), 8.50 (1 H, dd, J=2.64, 0.63 Hz), 7.74 (1 H, d, J=1.01 Hz), 7.47 (1 H, br. s.), 6.44 (1 H, dd, J=2.64, 1.64 Hz), 3.98 (1 H, d, J=9.57 Hz), 3.65 (1 H, d, J=9.57 Hz), 3.39 (1 H, dd, J=14.86, 1.51 Hz), 2.71 - 3.09 (5 H, m), 2.10 - 2.26 (2 H, m), 1.69 - 1.80 (1 H, m), 1.48 - 1.64 (2 H, m). LCMS: RT = 0.51 min, MH+ = 326.2.

## Claims

1. A compound selected from the group consisting of 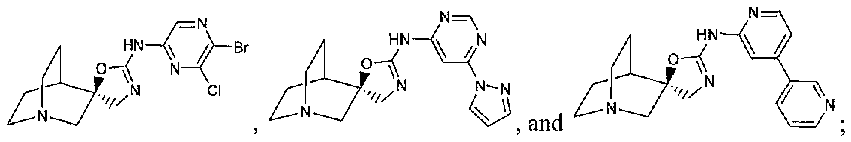 or a pharmaceutically acceptable salt thereof.

2. A pharmaceutical composition comprising a therapeutically effective amount of a compound of claim 1, or a pharmaceutically acceptable salt thereof, and a pharmaceutically acceptable carrier.

3. A compound of claim 1, or a pharmaceutically acceptable salt thereof for use in a method for the treatment of schizophrenia, Alzheimer's Disease, cognitive disorders, rheumatoid arthritis, osteoarthritis, ulcerative colitis, Crohn's Disease, or diabetes.

4. The compound for use according to claim 3 for the treatment of schizophrenia.

5. The compound for use according to claim 3 for the treatment of Alzheimer's disease.

## Patentansprüche

1. Verbindung, ausgewählt aus der Gruppe bestehend aus 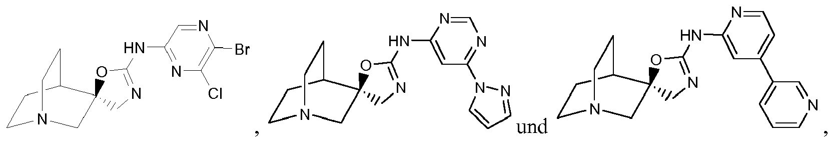 oder ein pharmazeutisch verträgliches Salz davon.

2. Pharmazeutische Zusammensetzung, umfassend eine therapeutisch wirksame Menge einer Verbindung nach Anspruch 1 oder ein pharmazeutisch verträgliches Salz davon und einen pharmazeutisch verträglichen Träger.

3. Verbindung nach Anspruch 1 oder ein pharmazeutisch verträgliches Salz davon zur Verwendung in einem Verfahren zur Behandlung von Schizophrenie, Alzheimer-Krankheit, kognitiven Störungen, rheumatoider Arthritis, Osteoarthritis, Colitis ulcerosa, Morbus Crohn oder Diabetes.

4. Verbindung zur Verwendung nach Anspruch 3 zur Behandlung von Schizophrenie.

5. Verbindung zur Verwendung nach Anspruch 3 zur Behandlung von Alzheimer-Krankheit.

## Revendications

1. Composé sélectionné à partir du groupe constitué de: 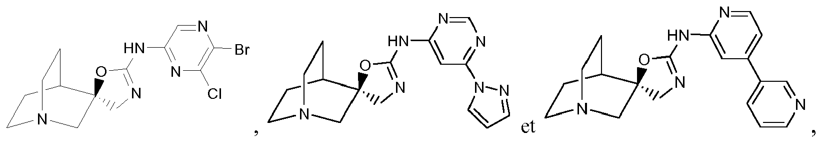 ou d'un de ses sels pharmaceutiquement acceptables.

2. Composition pharmaceutique comprenant une quantité pharmaceutiquement efficace d'un composé selon la revendication 1 ou un de ses sels pharmaceutiquement acceptables et un véhicule pharmaceutiquement acceptable.

3. Composé selon la revendication 1 ou un de ses sels pharmaceutiquement acceptables en vue d'une utilisation dans un procédé destiné au traitement de la schizophrénie, la maladie d'Alzheimer, de troubles cognitifs, de l'arthrite rhumatoïde, l'arthrose, de la colite ulcéreuse, la maladie de Crohn ou du diabète.

4. Composé en vue d'une utilisation selon la revendication 3 pour le traitement de la schizophrénie.

5. Composé en vue d'une utilisation selon la revendication 3 pour le traitement de la maladie d'Alzheimer.
